# EUROPEAN PATENT APPLICATION

(11) **EP 2 444 009 A1**
(43) Date of publication of application: **25.04.2012**
(21) Application number: 12150140.7
(22) Date of filing: 12.05.2006
(51) Int. Cl.: A61B 17/08, A61F 13/02

(54) **Dynamic tensioning system and method**

(30) Priority: 12.05.2005 US 68032405 P; 08.08.2005 US 70629705 P; 20.03.2006 US 78439906 P
(62) Divisional of application: 06759744.3
(71) Applicant: Canica Design Inc., Almonte, Ontario K0A-1AO (CA)
(72) Inventor: Archibald, Matthew, E., Stittsville, Ontario K2S 1H5 (CA); Rattew, Alden, Lombardy, Ontario K0G 1L0 (CA); Maxwell, Timothy, J., Stittsville, Ontario K2S 0A1 (CA); Lee, Leonard G., Almonte, Ontario K0A 1A0 (CA); Henderson, James, P., Gatineau, Québec J9H 5E1 (CA); O'Malley, Michael, T., Appleton, Ontario K0I 1A0 (CA); Barry, Julia, Van Eyk, Ottawa, Ontario K2C 2S9 (CA); Armstrong, David, W., Ottawa, Ontario K2B 5C7 (CA)
(74) Representative: Phillips & Leigh

(57) **Abstract**

Systems and methods of applying dynamic force to tissues to promote healing. Systems of this invention may be applied to tissue to bolster the tissues, move the tissues, or expand the tissue. Systems of this invention may also include a tension indication feature, allowing measured application of dynamic force to the tissue.

## Description

### Field of the Invention

This invention relates generally to a dynamic tensioning system and method, and specifically to a system for promoting healing of a tissue defect, either by stabilization of tissue, moving tissue or expanding tissue, as required.

### Background

Surgical procedures generally require modification of existing tissue. Healing after trauma may also necessitate changes in the tissues. Such modification may occur pre-surgery, or it may occur as a part of the post-surgical healing process. Successful healing methods may ideally include stable healing of incisions or wounds (post-surgery) and stretching of healthy skin (pre-surgery) to assist in post-surgery restorative measures, among others.

Pre or post surgical expansion of tissue is possible because plastic tissues, such as skin and muscle, possess certain viscous and elastic rheological properties, and are therefore viscoelastic. Certain plastic tissues are able to increase surface area over time, which can be termed "creep." "Mechanical creep" is the elongation of skin with a constant load over time, while "biological creep" refers to the generation of new tissue due to a chronic stretching force. A constant and unrelenting force applied to a body tissue, such as skin or muscle, may result in both mechanical and biological creep. Mechanical creep restores the tension originally present but lost in the skin across the incision or wound by retensioning skin or soft tissue cells, thereby increasing skin coverage. Biological creep occurs more slowly and involves the creation of new tissue. Pre-surgical tissue expansion has long been part of the art of plastic surgery, traditionally accomplished with balloon-type tissue expanders embedded under the skin and externally inflated and increased over time to create expanded pockets of skin for procedures such as breast reconstruction after radical mastectomies, and stretching healthy tissue prior to plastic surgery for the creation of flaps for soft tissue closure.

Pre-surgical tissue expansion may be indicated for repair of painful and unsightly past skin grafts, tumor excision, bums, or other such defects. Existing devices and procedures for expanding tissue, including skin, before surgery involve lengthy, costly and complicated processes. Multiple operations may be required before the chief surgical procedure may be performed. At minimum, multiple visits to a physician may be required, e.g. to further inflate a balloon. Complications can arise due to the invasive nature of existing procedures. Thus, there is a need for a device and system that safely and effectively provides an uncomplicated method for expanding tissue prior to surgery.

Post-surgery, it is often desirable to stabilize tissue, which can be complicated by increased internal volume; changes in aspect ratio, such as increased abdominal circumference created in prone, non-ambulatory patients due to muscular atrophy; respiratory muscular activity; muscular response; loss of fascia structure; muscular-skeletal deformation; and other complications. In addition, normal body movements such as breathing and sitting can cause discomfort and insecurity in a post-surgical patient, and can also cause tearing or re-opening of the wound. Such post-surgical complications compromise stable wound healing, extending the time required to heal following a procedure.

After primary closure of a wound or incision, adjunct systems may be used to bolster the reapproximation of the wound margins. For example, adhesive tissue glues, bond the wound edges together while adhesive tapes are placed across the incision and adhered to the skin. However, problems have been noted with the use of these existing products. Adhesive glues tend to result in poor wound edge approximation from glue leaking into the wound, resulting in widening of the scar. In addition, glues do not allow wound eversion, a critical element for optimal wound healing. Surgical adhesive tapes also discourage wound eversion because they adhere directly to the surface of the wound. Direct adherence to the surface of the wound may also rip open freshly healed tissues. These products are designed primarily to achieve static immobilization of underlying tissues. Therefore, a need exists for a system to bolster and stabilize primarily closed wounds.

At times wounds do not follow normal healing stages, and are labeled chronic wounds. A chronic wound may be caused by a variety of factors, including infection, tissue hypoxia, diabetes mellitus, malnutrition, or immunodeficiency. These wounds are difficult to manage and are resistant to normal post-surgery healing routines. Such wounds present a variety of quality of life issues. Thus, a need exists for a system to promote healing of chronic wounds.

### Summary

Embodiments of this invention include a dynamic tensioning device having a dynamic component, anchoring elements and a limiting element. The dynamic element may be a dynamic membrane that applies tension to the tissues. The limiting element may be a non-elastic clear film layer located on top of and not attached to the dynamic element. The limiting element establishes correct tension upon application of the device, limits dehiscence caused by edema, body movement and coughing, and prevents tensioned tissue from retracting or re-opening. A dynamic tensioning device of this invention may be provided in various sizes to accommodate a variety of applications, as described below.

Embodiments of this invention provide a dynamic tensioning system that bridges and stabilizes a wound or incision to isolate the wound or incision and to reduce pain and discomfort. Such systems reinforce primarily closed skin defects and may also provide primary closure force for small skin defects, such as a facial mole or melanoma excision. Such systems also provide force for stretching or pulling skin edges together, such as in the preparation for eventual surgical closure of a cleft of an upper lip.

Systems of this invention may also be applied to chronic wounds. Application on a chronic wound provides dynamic force on opposite sides of the wound to pull wound edges together, reducing the size of and ultimately closing the defect.

Additional embodiments provide a dynamic tensioning system that stretches healthy skin and encourages growth of new skin. Such systems may be applied in preparation for a surgical procedure or post-surgery. A dynamic tensioning system of this invention provides dynamic force on opposite sides of a wound, or in a desired area of tissue expansion. In this embodiment, the stretch limiting element prevents over-tensioning during installation, and is discarded after installation is accomplished.

Another system of this invention allows measurement of applied tensions. Repositionable adhesive permits re-tensioning as needed and as indicated by guide marks on the device.

In summary, among other things, this invention is: a system and method for stabilization of a wound or incision, a system and method for stretching healthy tissue for expansion of the tissue, a system and method that promote healing of chronic wounds, and a system and method for measured application and adjustment of a dynamic force to tissue.

Embodiments of this invention include a device for applying force to a patient's tissue, the device comprising: two anchoring elements attachable to the tissue; an elastic element attached to each of the anchoring elements; and a limiting element that limits extension of the elastic element.

An alternative embodiment of this invention includes a device for applying force to a patient's tissue, the device comprising: first and second anchoring elements attachable to the tissue; an elastic component comprising two ends, wherein a first end of the elastic component is attached to the first anchoring element; a non-elastic member comprising a marker and two ends, wherein the first end of attached to the second end of the elastic component and wherein the second end is releasably attached to the second anchoring element; and an indicator element comprising first and second indicators, wherein the indicator element is coupled to the first anchoring element.

Methods of this invention include a method of applying dynamic tension to tissues using a device comprising two anchoring elements attachable to the tissue, a release liner, a dynamic component attached to each of the anchoring elements, and a limiting element attached to each of the anchoring elements and that limits extension of the dynamic component, the method comprising: removing a portion of the release liner of the device, applying force in opposite directions to load the dynamic component until further separation is limited by the limiting element; securing the anchor element to the tissue; removing the two remaining segments of the release liner; and pressing on the anchoring elements to secure the adhesive to the tissue.

Additional methods include a method of expanding healthy tissue using a device comprising two anchoring elements attachable to the tissue, a release liner, a dynamic component attached to each of the anchoring elements, and a limiting element attached to each of the anchoring elements and that limits extension of the dynamic component, the method comprising: establishing an elliptical shape having a 3:1 length to width ratio around a defect that is to be excised; installing devices over the elliptical shape, starting at the top and bottom portions of the shape, the installation comprising: removing a portion of the release liner of the device, applying force in opposite directions to load the dynamic component until further separation is limited by the limiting element; applying the device to the tissue; removing the two remaining segments of the release liner; and pressing slightly on the anchoring elements; as the devices become untensioned due to skin expansion, removing the applied devices and replacing them with new tensioned devices to continue stretching; and continuing to replace untensioned devices until opposing lines of the ellipse meet.

Methods of this invention include a method of measuring and adjusting dynamic tension applied to tissue using a device comprising: first and second anchoring elements; a release liner; an elastic component comprising two ends, wherein a first end of the elastic component is attached to the first anchoring element; a non-elastic member comprising two ends, wherein the first end is attached to the second end of the elastic component, and wherein the second end of the non-elastic member is releasably attached to the second anchoring element; a marker; and an indicator element comprising first and second indicators, wherein the indicator element is attached to the first anchoring element; the method comprising: removing a center portion of the release liner; applying the device to tissue with the first indicator aligned with the marker; removing the remainder of release liner; pressing the device to achieve good adhesion; pulling the non-elastic member to stretch the elastic component until the marker is aligned with the second indicator; and when the tissue movement causes the marker to align with the first indicator, releasing the non-elastic member and repositioning it on the anchor element to re-align the marker with the second indicator.

### Brief Description of the Drawings

Figure 1 is a perspective view of a dynamic tensioning device of this invention, illustrated in a relaxed condition.
Figure 2 is a side view of the device of Figure 1.
Figure 3 illustrates removal of the center portion of the release liner of the device of Figure 1.
Figure 4 is a perspective view of the dynamic tensioning device of Figure 1, illustrated in a tensioned condition.
Figure 5 illustrates removal of the remaining portions of the release liner upon installation of the device of Figure 1.
Figure 6 is a perspective view of an installed system of this invention, as illustrated in Figures 1-5.
Figure 7 is a perspective view of an alternative embodiment of this invention with a perforated tension limiting film.
Figure 8 is a perspective view of the device of Figure 7, illustrated in a tensioned condition.
Figure 9 is a perspective view of a dynamic tensioning device according to an alternative embodiment of this invention and illustrated in an untensioned condition.
Figure 10 illustrates removal of the center portion of the release liner of the device of Figure 9.
Figure 11 is a perspective view of the dynamic tensioning device of Figure 9, illustrated in a tensioned condition.
Figure 12 illustrates removal of another portion of the release liner of the device of Figure 9.
Figure 13 is a perspective view of a system of this invention.
Figure 14 illustrates an embodiment of a tensioning system having replaceable dynamic components.
Figure 15 illustrates an embodiment of a system of this invention used to provide closure force to an open wound.
Figure 16 is an illustration of preparation of tissue for application of a system of this invention.
Figure 17 is a top plan view of an embodiment of a system of this invention.
Figure 18 is a top plan view of tissue treated with the system of Figure 16, illustrating results of tissue movement after application of the system.
Figure 19 is a perspective view of an embodiment of a system of this invention applied to promote healing of a chronic wound.
Figure 20 is an exploded perspective view of an embodiment of a tension measuring device of this invention.
Figures 21-23 illustrate use of the tension measuring device of Figure 19.
Figure 24 is an exploded perspective view of a dynamic tensioning device of an embodiment of this invention having a removable limiting element.
Figure 25 is a partially exploded perspective view of the device of Figure 24.

### Detailed Description

Embodiments of this invention provide systems for applying dynamic force to tissues. Devices of this invention may be applied to tissue to promote tissue healing, bolster the tissues, move the tissues, or expand the tissue. Devices of this invention may also include a tension indication feature, allowing measured application and adjustment of dynamic force to the tissue.

### Dynamic Tensioning Device

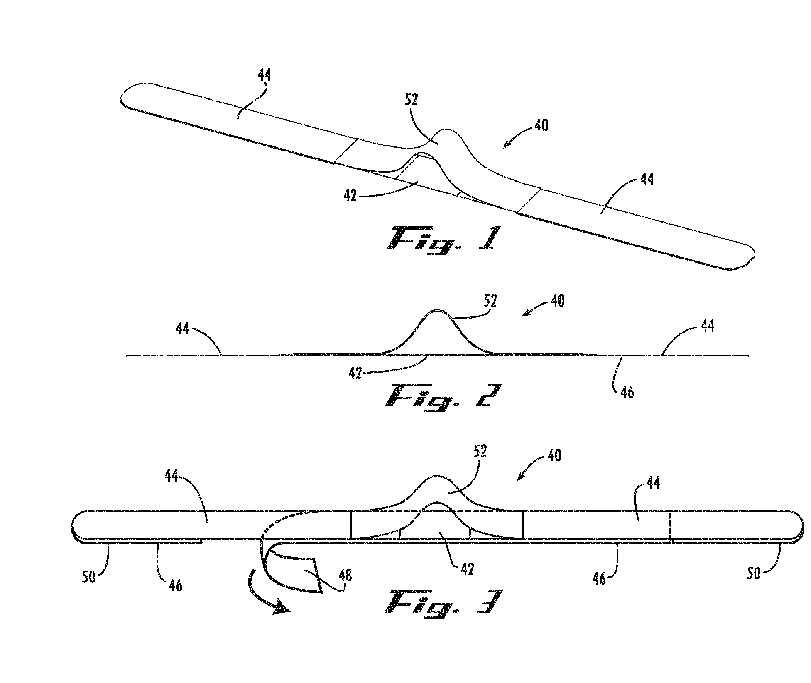

Embodiments of this invention include a device having anchoring elements, a limiting element, and a dynamic membrane. In one embodiment, shown in Figures 1-6, tensioning device 40 includes a dynamic membrane 42 located between anchor elements 44. Anchor elements 44 attach the device 40 to the tissue. Anchor elements 44 may be formed from breathable skin tape or adhesive fabric. Such materials include 1529 Micropore® rayon, available from 3M, as well as additional materials, including woven or non-woven fabrics, rayon or polyester blends, polyester, coated plastic films that are suitable for heat-sealing or ultrasonic welding, or any other suitable film, fabric or other material.

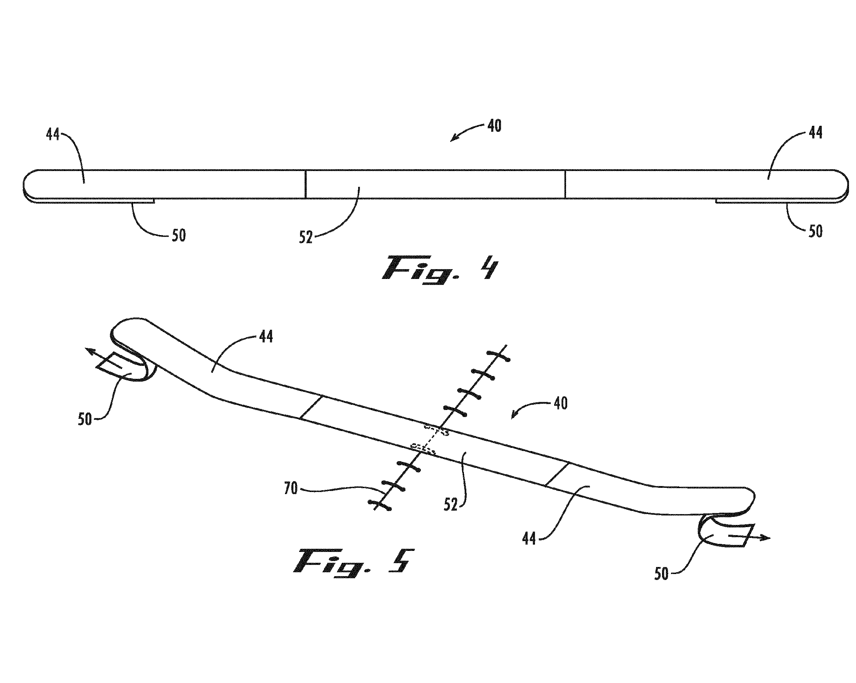

Release liner 46 is releasably attached to the bottom side of anchor elements 44. Liner 46 includes center portion 48 and end portions 50 (shown in Figures 3 and 5), which are easily peeled away from device 40. Release liner 46 may be formed from paper, wax paper, film or acetate or any other suitable material.

In one embodiment, dynamic membrane 42 is relatively transparent, allowing visualization of the wound through that membrane. The membrane may or may not be entirely clear. Dynamic membrane 42 may be stretched as the device 40 is attached to the tissue, and upon installation, apply dynamic force to the tissue. Dynamic membrane 42 may be made from an elastic material such as silicone or thermoplastic elastomer (TPE) or from any other suitable material.

In order to monitor and control the amount of tension applied, devices of this invention may incorporate a limiting element 52 having controllable ranges and upper limits for therapeutically beneficial dynamic tensions. In this manner, the limiting element 52 establishes the amount of tension applied by the device 40. Limiting element 52 may be formed from rigid, non-elastic polyester, or any other suitable material.

As shown in Figures 1, 2 and 6, when the device 40 is in a relaxed, untensioned condition, limiting element 52 is not extended with the result that a loop of limiting element 52 extends up and away from dynamic membrane 42. Limiting element 52 limits dehiscence (splitting open or rupture) caused by edema, body movement, coughing, and other such factors. By preventing the tensioned tissue from retracting and re-opening, the limiting element 52 provides a safety mechanism to protect the wound or incision from re-opening.

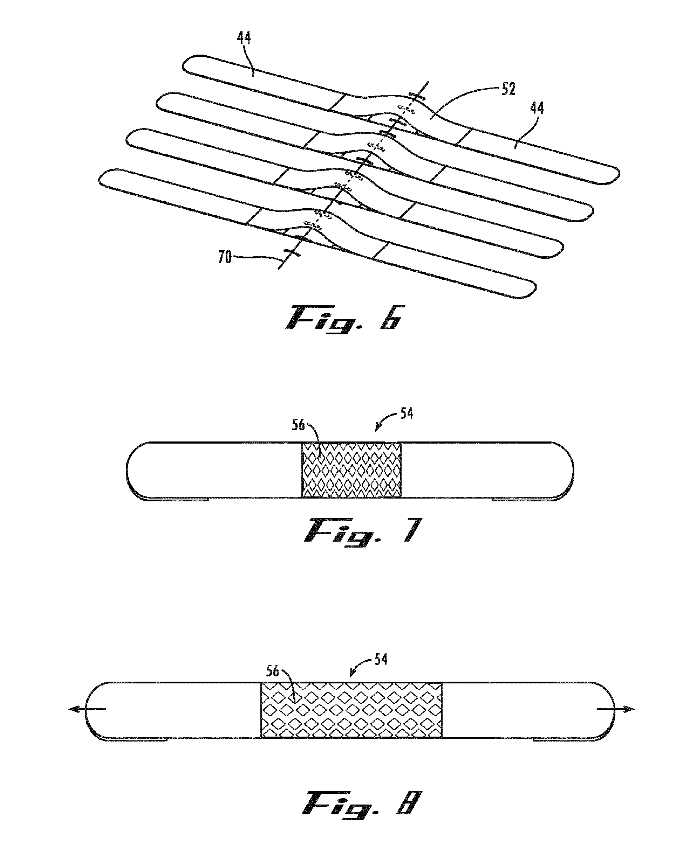

In an alternative embodiment, illustrated in Figures 7-8, dynamic tensioning device 54 includes a limiting element 56 formed from a diamond mesh. The mesh limiting element 56 has a predetermined elastic limit. Use of the mesh eliminates the looping upward of the stretch limiting element 53, shown in Figures 1-3. In addition, mesh allows the wound to breathe, and provides excellent visualization of the wound or incision.

In another embodiment, the limiting element is an integral part of the dynamic component. For example, the dynamic membrane may include filaments that limit stretching, such as relatively rigid elements having accordion-like folds, or other suitable filaments. In another embodiment, a mesh limiting element, similar to the mesh described above, may be incorporated into the dynamic component. The limiting element may be formed from any suitable biologically inert material.

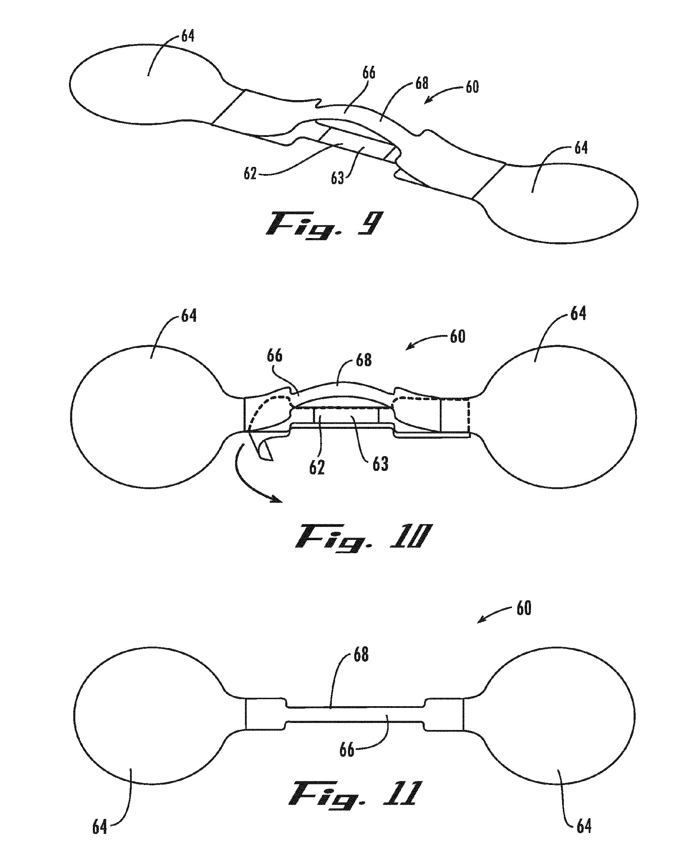

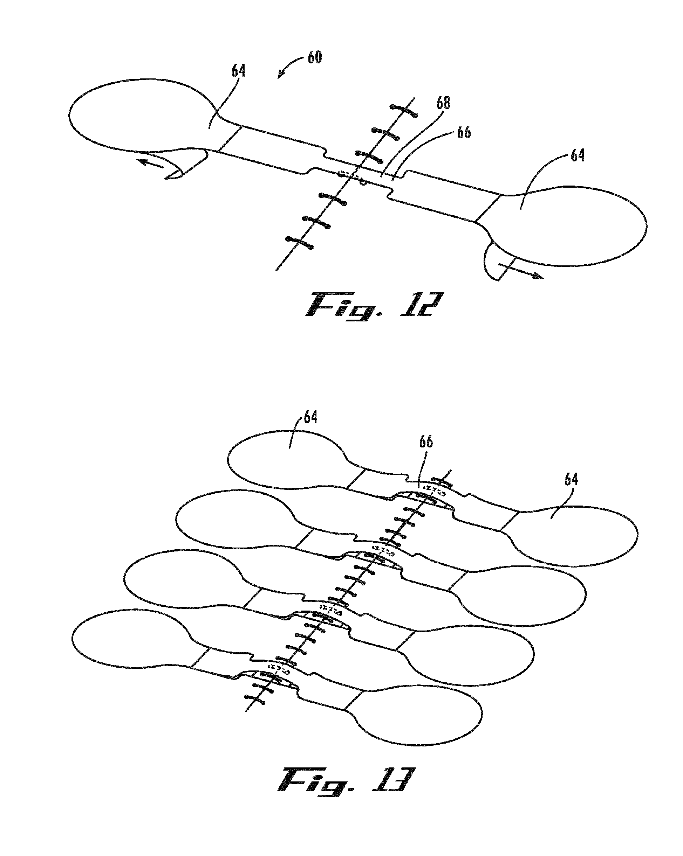

In an alternative embodiment of this invention, shown in Figures 9-13, tensioning device 60 includes a dynamic membrane 62 located between anchor elements 64. Anchor elements 64 attach the device 60 to the tissue as described above. Dynamic membrane 62 includes narrow portion 63. Limiting element 66 also includes a narrow section 68. These narrower portions permit use of the device in smaller areas, such as the cleft lip of a baby.

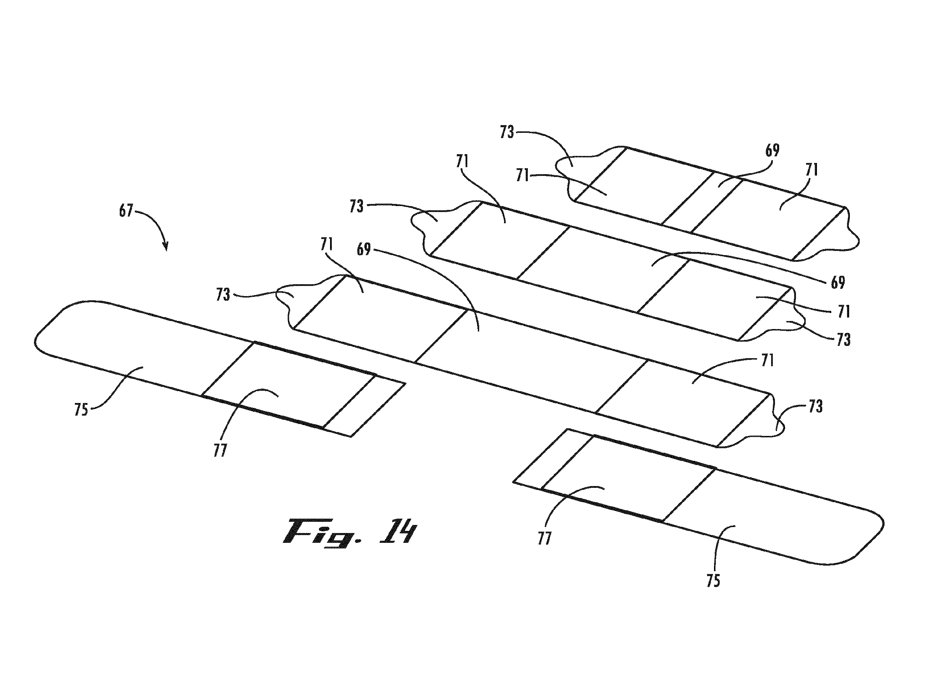

In another embodiment, shown in Figure 14, the dynamic tensioning device 67 includes removable dynamic components 69. Dynamic component 69 may be removed and replaced by shorter components 69 to increase tensions applied as closure of a wound progresses. Ends 71 of dynamic component 69 are releasably attached to anchor elements 75, and include tabs 73 for easy lifting and removal. Ends 71 of dynamic component 69 may be attached to the anchor elements 75 using releasable adhesive 77, or by any other suitable means. This replacement dynamic component system may be used with or without a limiting element. In this embodiment, the anchor elements 75 remain attached to the tissue, and the dynamic component 69 is replaced as needed during closure of the wound or defect. This embodiment may also be used to expand tissues.

Components of devices of this invention may be assembled and joined using adhesives, by heat sealing the components together, or by any other suitable method of bonding the components. For example, the anchoring elements, dynamic membrane, and limiting element of the embodiments described above may be joined by adhesives, or may be formed from materials that allow the elements to be heat sealed.

The devices described above may be manufactured in a variety of sizes used in various applications of the system, as further described below. For example, a longer membrane and limiting element may be required to span the width of a larger wound or incision, while a smaller device is more useful for application to a facial or other small defect. Devices of this invention are useful to bolster primarily closed incisions, deliver closure force to an open wound, stretch healthy tissue and promote healing of chronic wounds, along with other uses.

### Bolstering Incision and Wound Closure

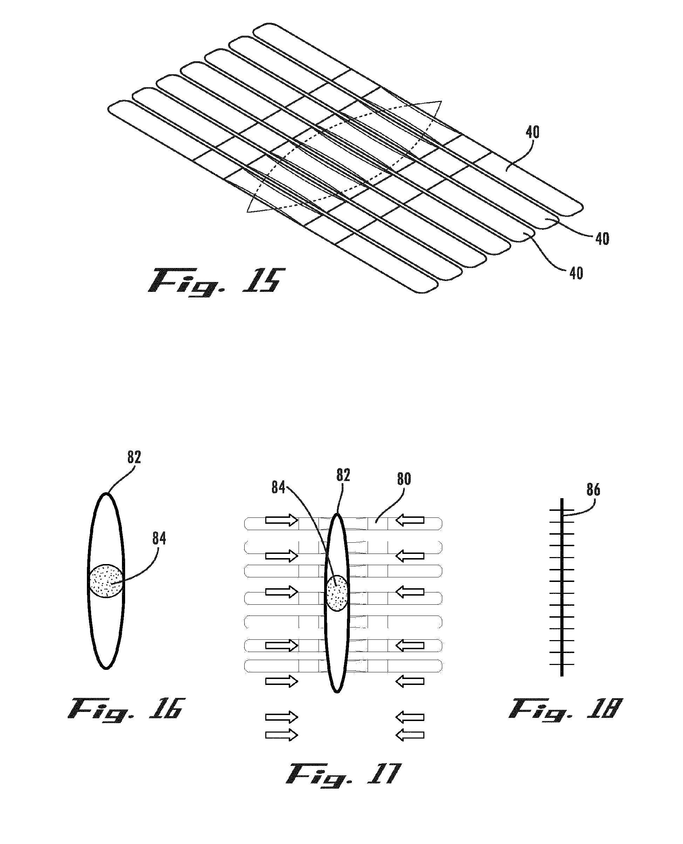

Installation of dynamic tensioning devices of this invention across a primarily closed incision bridges and stabilizes the wound and reduces incision pain. In addition, the tension applied by devices of this invention provides closure force to pull wound edges toward one another. In one embodiment, a system is applied as a wound closure system, as illustrated in Figure 15.

Applying one or more devices to a wound or incision increases patient comfort during activities or at rest, encourages mobility by isolating the area from body movement, and does not interfere with application of medications to the wound or incision or with visualization of the wound or incision.

To attach device 40, shown in Figures 1-6, to the skin of a patient, the skin is shaved and cleaned in an area extending the width of the device from both sides of a line defining the defect, i.e. the wound or incision. Skin preparations, such as Compound Benzoin Tincture, may be used and enhance adhesion of the device to the skin. As shown in Figure 3, center 48 of the release liner 46 is removed first. The device 40 is stretched until the limiting element 52 becomes taut, as shown in Figure 4. In this manner, force is applied in opposite directions to load the dynamic component until further separation is limited by the limiting element. Center 48 of device 40 is then aligned with the defect line 70 (Figures 5 and 6), and both sides of device 40 are applied at the same time. The remainder of the release liner 46 is then removed (Figure 5) and the adhesive fabric is pressed slightly to ensure good adhesion to the skin. As shown in Figure 6, multiple devices 40 may be applied across an incision 70 to bolster staples or sutures. Smaller sizes of devices 40 may also be installed to bolster or close smaller defects, such as excision of a facial mole or melanoma.

### Tissue Expansion

Embodiments of this invention may be used to expand tissue prior to surgery. For example, device 60, shown in Figures 9-13, may be used to expand tissue when a device having a narrow width is more appropriate, such as in the closure and healing of a cleft lip and or palate of a newborn baby. Oral-facial clefts are some of the most common major birth defects and refer to a condition in which segments of the palate or lip are not properly fused. Such defects may include a cleft lip, cleft palate, or both. In one embodiment of this invention, shown in Figures 9-13, device 60 is applied over a cleft lip so that the device 60 exerts constant, gentle force on the segments of the lip to pull them together prior to surgery.

Device 60 is easily applied to a baby's lip and cheeks due to the size of narrow portions 63 and 68. Dynamic tension is thereby applied to the baby's lips and cheeks, pulling the sections of the cleft lip toward one another. In this manner, the tissue is conditioned for the procedure that will surgically fuse the two segments. This prior conditioning will help to reduce the point loading on sutures during and after the surgical procedure. In addition, device 60 may be applied post-surgery to bolster the sutures. In both applications, limiting element 66 maintains proper closure even if the baby cries, yawns or otherwise moves the facial muscles in a manner that may otherwise disrupt the healing cleft.

Another embodiment, shown in Figures 16-18, may also be used to expand healthy skin. Using a device 80 (similar to device 40 described above) skin may be expanded prior to a surgical procedure. In one embodiment, illustrated in Figure 16, prior to surgery, the physician traces an elliptical shape 82 having a 3:1 length to width ratio around the defect 84 that is to be excised. As shown in Figure 17, devices 80 are then installed over the elliptical shape 82, starting at the top and bottom portions of the shape 82. The elliptical shape 82 slowly narrows as the skin expands. As the devices 80 become untensioned due to skin expansion, the applied devices are removed and replaced with new tensioned devices to continue stretching. When the skin is stretched such that opposing lines of the ellipse meet, the surgical procedure is performed to remove the defect 84. The wound is closed as a clean linear incision 86, as shown in Figure 18.

This method and device may also be used in any situation in which skin expansion is desired, such as the removal of skin defects, including melanoma, existing keloid scars, and other defects. In situations in which skin is being stretched for reasons other than removal of a defect, the surgeon may draw an ellipse having a 3:1 ratio to the size of the additional skin needed.

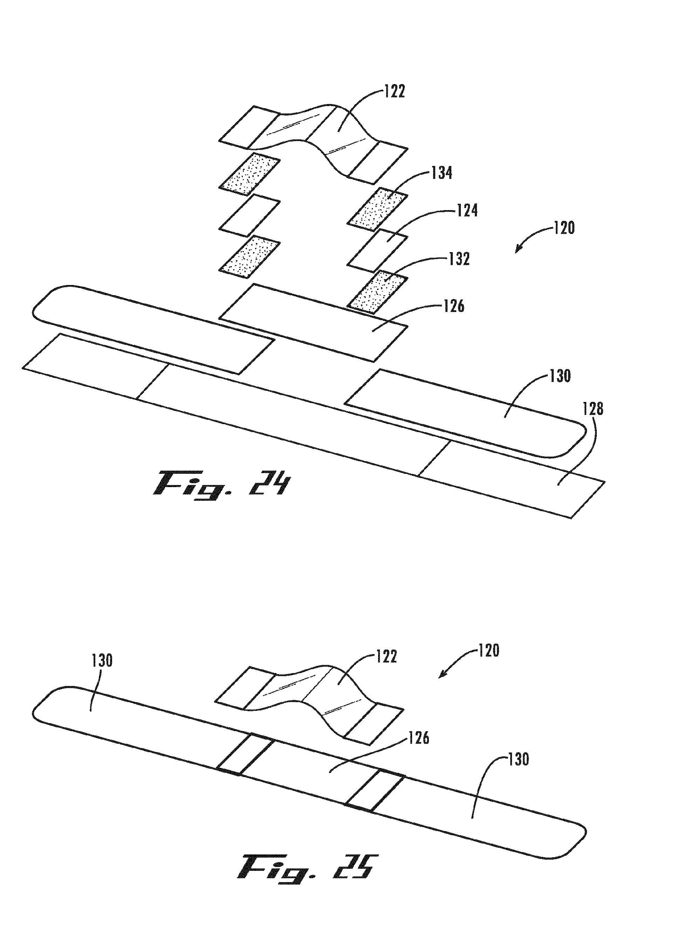

In some embodiments, the limiting element may be removed after the tensioning device is installed. As shown in Figures 24-25, once the device 120 is applied in tension to the skin, the removable limiting element 122 is removed by peeling it from the release film 124 that is on either end of the dynamic component 126. As shown in Figure 24, anchor release liner 128 is attached to anchor elements 130. Dynamic component 126 may be heat-sealable to the anchor elements 130. Adhesive 132 bonds release liner 124 to dynamic component 126, and adhesive 134 is attached to release liner 124 of limiting element 122. This embodiment may be used in any application in which the limiting element is not required after installation of the device, including tissue expansion prior to surgery and chronic wound care.

### Chronic Wound Care

Embodiments of this invention may also be used to promote healing of chronic wounds by restoring normal skin tension at the wound site. The restoration of normal skin tension at the wound site migrates skin over the defect to provide viable skin coverage or reduce the defect size. Additionally, application of this system to a chronic wound provides increased vascularity, allows radial reduction of the wound site, (because the devices are applied all around the wound and pull in towards the center of the wound), provides clear visibility of wound since the materials that bridge the wound are clear, affords moisture retention due to materials used (such as silicone elastomer), reduces edema (swelling), and reduces pain by reducing tension. In addition, devices of this invention are easy for the physician and patient to install, and with regular supervision by the physician, patients can manage the device at home - an important feature allowing a patient a more normal course of healing.

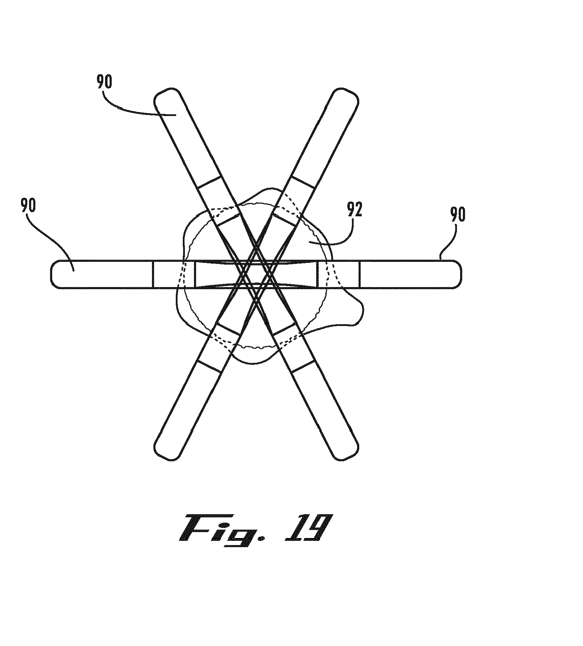

As shown in Figure 19, device 90 (similar to devices 40 and 80, described above) may be installed around a generally circular wound 92, or any other irregularly shaped defect, providing tension from various directions. As in other embodiments, a limiting element is used to appropriately apply the tensioned device. However, the limiting element may be attached with removable adhesive and may be removed from the device 90 after application to the patient, as described above. Removal of the limiting element allows multiple devices 90 to be overlapped. In an alternative embodiment, the anchor elements may have a triangular shape, or any other shape that allows the devices to be installed such that the anchor elements do not overlap.

### Tension Indicator System

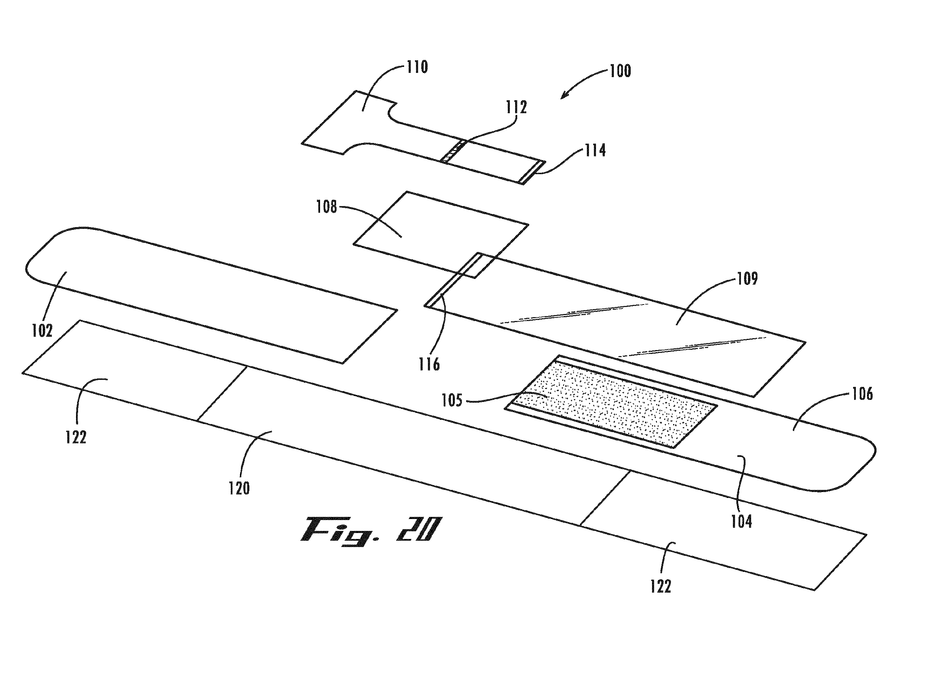

Another embodiment of this invention, shown in Figures 20-23, provides a tension indicator element that allows measured adjustment of applied tension. As shown in Figure 20, dynamic tensioning device 100 includes anchoring elements 102, 104, both of which are attached to the skin using adhesive and release liner 122. Release liner 122 may be an easy to peel release liner that can take a crack-back cut. Anchoring elements 102, 104 may be formed from a breathable, conformable, printable, heat-sealable irradiation safe skin tape, or from any other suitable material. Second anchoring element 104 includes repositionable adhesive 105 on the top surface 106, allowing releasable attachment of film 109, which is attached to dynamic component 108. In one embodiment, adhesive 105 is permanently bonded to second anchoring element 104, an provides strong sheer strength to film 109, while allowing film 109 to be easily peeled off and reattached numerous times. This releasable attachment allows adjustment of the tension of the device 100 without repeated repositioning of the anchor elements 102, 104, which directly contact the patient's skin, which repositioning could reduce the adhering capability of the adhesive.

Film 109 may be a clear release liner that is printable on one side and that provides strong sheer and weak peel forces when bonded to adhesive 105. Film 109 may be non-elastic member, or may be any other suitable material, and may be permanently heat-sealed or adhered to dynamic component 108. Film 109 includes marker 116 (a transverse line on component 109), used to set and adjust the tension, as described below. In an alternative embodiment, marker 116 may be located on the dynamic component, or on any other suitable portion of the device.

An indicator element, such as indicator flap 110, is attached to first anchoring element 102 and includes first indicator 112 and second indicator 114, shown here as transverse lines on indicator flap 110. First and second indicators 112, 114 may be different colors so that they are easily distinguished, or may be set apart in any other suitable manner.

To install the device 100, center portion 118 of release liner 120 is removed and device 100 is applied to the tissue, with the first indicator 112 aligned with marker 116 of film 109. Then, the ends 122 of release liner 120 are removed and anchor elements 102, 104 are pressed to ensure good adhesion to the tissue.

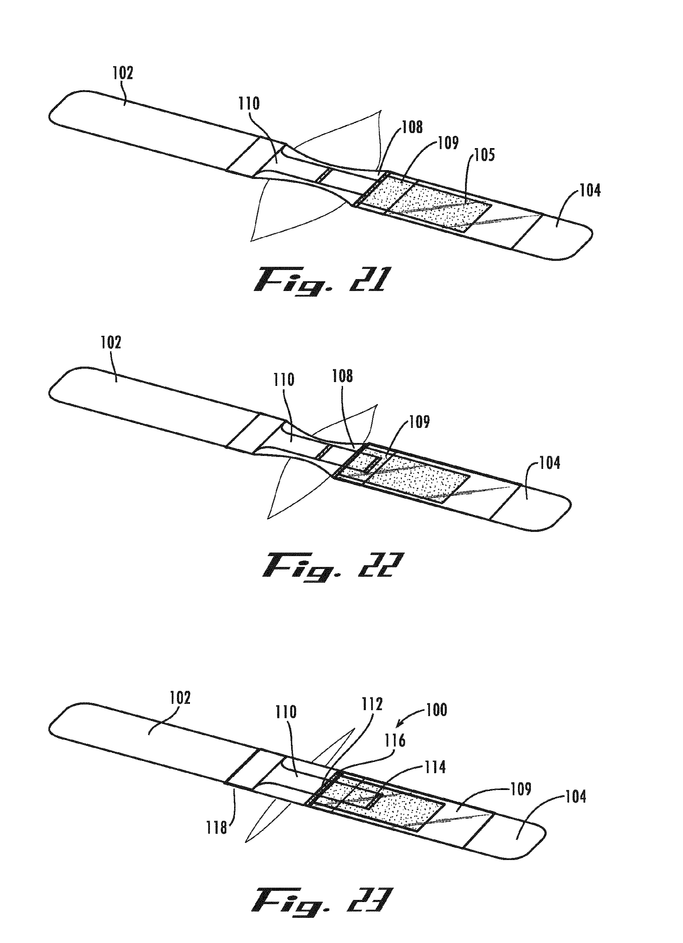

As shown in Figures 21-23, after the device is installed, to apply tension to the tissue, film 109 is pulled so that dynamic component 108 is stretched until marker 116 is aligned with second indicator 114. Tension is thereby applied to pull the wound edges toward one another, as shown in the Figures. When marker 116 is aligned with first indicator 112, tension is no longer being applied. To re-set the tension, film 109 is released from adhesive 105, and marker 116 is re-aligned with second indicator 114. In this manner, dynamic component 108 is stretched to apply tension to the tissues. Resetting the tension applies set closure force and again pulls the edges of the defect toward each other.

The tension indicator device described may be used with other systems described above for closure of wounds, to stretch tissue, and to promote healing of chronic wounds. The tension indication feature may be used in place of the limiting element in any application in which measurement of tension is desired and the limitation of stretch is not the primary concern.

While the invention has been described in detail with particular reference to the disclosed embodiments, it will be understood that variations and modifications can be affected within the spirit and scope of the invention as described herein.

### The following features were claimed in the parent application and are appended here to provide basis for amendment should such be required:

1. A device for applying force to a patient's tissue, the device comprising:
   (a) two anchoring elements attachable to the tissue;
   (b) an elastic element attached to each of the anchoring elements; and
   (c) a limiting element that limits extension of the elastic element.
2. The device of feature 1, wherein the elastic element is relatively transparent.
3. The device of feature 1, wherein the elastic element is removably attached to each of the anchoring elements and the device further comprises at least one additional elastic element.
4. The device of feature 1, wherein the limiting element is a mesh material.
5. The device of feature 1, wherein the limiting element is a membrane.
6. The device of feature 1, wherein the elastic element is a membrane comprising filaments that act as the limiting element when stretched.
7. The device of feature 1, wherein the limiting element is releasably attached to each of the anchoring elements.
8. The device of feature 1, wherein the anchoring elements are attached to the tissue by adhesive.
9. The device of feature 1, further comprising a release liner having three segments.
10. The device of feature 1, wherein the elastic element and the limiting element each further comprise a narrow section.
11. The device of feature 1, wherein the anchor elements each further comprise an enlarged end.

## Claims

1. A device for applying force to a patient's tissue, the device comprising:
(a) first and second anchoring elements attachable to the tissue;
(b) an elastic component comprising two ends, wherein a first end of the elastic component is attached to the first anchoring element;
(c) a non-elastic member comprising a marker and two ends, wherein the first end is attached to the second end of the elastic component and wherein the second end is releasably attached to the second anchoring element; and
(d) an indicator element comprising first and second indicators, wherein the indicator element is coupled to the first anchoring element.

2. The device of claim 1, wherein the elastic component is relatively transparent.

3. The device of claim 1, wherein the anchoring elements are attached to the tissue using adhesive.

4. The device of claim 1, further comprising a release liner having three segments.

5. The device of claim 1, wherein the non-elastic element is a film.
